# EUROPÄISCHE PATENTSCHRIFT

(11) **EP 1 905 386 B1**
(45) Veröffentlichungstag und Bekanntmachung des Hinweises auf die Patenterteilung: **15.06.2011**
(21) Anmeldenummer: 06020639.8
(22) Anmeldetag: 29.09.2006
(51) Int. Cl.: A61F 2/16

(54) **Vorrichtung zum Falten oder Rollen einer in ein Auge zu implantierenden Intraokularlinse**
Device for folding or curling an intraocular lens to be implanted in an eye
Dispositif de pliage ou d'enroulement d'une lentille intraoculaire à être implantée dans un oeil

(43) Veröffentlichungstag der Anmeldung: 02.04.2008
(73) Patentinhaber: *Acri. Tec GmbH, 16761 Hennigsdorf (DE)
(72) Erfinder: Pankin, Dmitry, 13355 Berlin (DE); Kreiner, Hans-Jürg, Dr.rer.nat., 81545 München (DE)
(74) Vertreter: Eisenführ, Speiser & Partner

(56) Entgegenhaltungen:
- EP-A1- 1 114 623
- EP-A1- 1 466 571
- WO-A-99/29267
- WO-A-2005/082285
- WO-A2-03/044946
- US-A1- 2002 103 490

## Beschreibung

Die Erfindung betrifft eine Vorrichtung zum Falten oder Rollen einer in ein Auge zu implantierenden Intraokularlinse nach dem Oberbegriff des Patentanspruches 1.

Eine derartige aus DE 101 64 420 A1 bekannte Vorrichtung beinhaltet zwei schwenkbar miteinander verbundene Halbschalen, die von einer offenen Ausgangsstellung zur Aufnahme der ungefalteten Linse in eine geschlossene Endstellung zum Falten oder. Rollen der Linse geschwenkt werden können. Das Linsenaufnahmefach bildet in dieser geschlossenen Stellung einen Führungskanal, welcher in Implantationsrichtung an seinen beiden Enden offen ist und aus welchem die zu implantierende Linse durch eine in ein Auge einzuführende Injektorkanüle mit Hilfe eines Injektorstößels in das Auge implantiert werden kann. Zum Halten der Intraokularlinse, insbesondere beim Falten ist eine folienartige Bandschlaufe vorgesehen, die mit ihrem einen Ende an einer der beiden Halbschalen befestigt ist und an der zweiten Halbschale verschieblich geführt wird. In der offenen Ausgangsstellung wird zwischen der Bandschlaufe und den beiden Halbschalen der Aufnahmeraum für die Intraokularlinse gebildet. Zur Erleichterung der Faltbewegung, d.h. zum Bewegen der beiden Halbschalen von der offenen Ausgangsstellung in die Endstellung, sind an die Halbschalen Flügel, welche von Hand betätigt werden können, vorgesehen.

Auch bei der aus US 4,681,102 bekannten Vorrichtung sind an den beiden Halbschalen, welche das Linsenaufnahmefach bilden, Betätigungsflügel vorgesehen, um die Halbschalen aus der offenen Ausgangsstellung in die geschlossene Endstellung zu verschwenken. Auch diese bekannte Vorrichtung wird als Kartusche in einem Injektor verwendet, mit welchem die gefaltete Linse mit Hilfe eines Stößels durch eine Injektorkanüle in das Auge implantiert wird. Bei dieser bekannten Vorrichtung fehlt eine Halteeinrichtung, so dass beim Falten der Linse die Gefahr besteht, dass diese aufgrund ihrer federelastischen Eigenschaften unzureichend zwischen den Halbschalen gehalten wird und sich die Linse selbsttätig aus dem offenen oder noch teilweise offenen Linsenaufnahmefach entfernt.

Die Druckschrift WO 99/29267 offenbart für eine Intraokularlinse (IOL) eine Faltvorrichtung und einen IOL-Injektor sowie ein Verfahren zum Halten einer IOL bzw. zum Laden einer IOL in einen IOL-Injektor, was einem Arzt die Möglichkeit eröffnet, zwischen zwei Vorgehensweisen zu wählen, nämlich dem direkten Beladen oder dem Benutzen eines Operationsinstruments wie z. B. einer Pinzette. Hierzu ist die Faltvorrichtung je nach gewählter Vorgehensweise in eine von zwei vorgesehenen Faltrichtungen zu falten.

Aufgabe der Erfindung ist es eine Vorrichtung der eingangs genannten Art zu schaffen, bei welcher ein vereinfachtes Laden der Intraokularlinse in das offene Linsenaufnahmefach erreicht wird, wobei die im Linsenaufnahmefach angeordnete Linse bei offenem Linsenaufnahmefach und beim Falten sicher positioniert ist.

Diese Aufgabe wird erfindungsgemäß durch die Merkmale des Patentanspruches 1 gelöst, wobei in den Unteransprüchen vorteilhafte Weiterbildungen der Erfindung enthalten sind.

Bei der Erfindung beinhaltet die Halteeinrichtung zwei Halteelemente, welche an den beiden schwenkbar miteinander verbundenen Linsenaufnahmeteilen befestigt sind. An den Halteelementen sind jeweils Anschlagsflächen vorgesehen, welche diametralen Randbereichen der im offenen Linsenaufnahmefach liegenden, ungefalteten Linse gegenüber liegen und eine Bewegung der Linse aus dem Linsenaufnahmefach verhindern. Bei geschlossenem Aufnahmefach sind die Anschlagflächen in solche Positionen bewegt, dass sie mit den von den Linsenaufnahmeteilen gebildeten Begrenzungsflächen das Linsenaufnahmefach vorzugsweise bündig umschließen. Die im geschlossenen Linsenaufnahmefach vorhandene Intraokularlinse ist gefaltet oder gerollt. Die Intraokularlinse befindet sich im gefalteten oder gerollten Zustand in einer genau definierten Position, die reproduzierbar ist. Damit wird gewährleistet, dass die Linse bei der Implantation mit einer gewünschten Position aus dem Linsenaufnahmefach in das Auge gebracht werden kann. Das Linsenmaterial kann in einen solchen Zustand komprimiert sein, dass es für den Vortrieb in Implantationsrichtung insbesondere im Berührungsbereich zwischen Vortriebsstößel und Linse eine erhöhte Steifigkeit aufweist. Hierdurch ist es möglich, auch durch sehr kleine Inzisionen von z.B. 1,5 mm bis 1,9 mm die Implantation mit Hilfe eines Injektors in das Auge durchzuführen. Demzufolge kommt die Erfindung vorzugsweise bei elastischen Linsen vom MICS (Micro Incision Cataract Surgery) Typ zum Einsatz. In bekannter Weise kann zur Erleichterung der Vortriebsbewegung der Linse eine die Gleitfähigkeit verbessernde viskoelastische Lösung verwendet werden.

Vorzugsweise wird beim Verschwenken der Linsenaufnahmeteile ein von den Halteelementen erzeugter Druck an den Anschlagflächen auf die diametralen Randbereiche der Linse ausgeübt, wodurch die zunächst locker liegende Intraokularlinse seitlich zur Linsenmitte hin gestaucht wird.

Ferner können an den Anschlagflächen innenliegende Kanten ausgebildet sein, welche im Wesentlichen parallel zur Längsmitte der zu faltenden Linse verlaufen. Diese innen liegenden Kanten haben jeweils einen bestimmten Abstand zu den diametralen Rändern der Linse, welcher etwa 1 mm betragen kann. Beim Aufeinanderzuschwenken der Linsenaufnahmeteile wird von diesen Kanten auf die Linse eine in Richtung zum Boden oder in das Innere des Linsenaufnahmefachs gerichteter Druck ausgeübt, so dass durch die dabei auf den Linsenkörper einwirkenden Momente der Faltvorgang um die Längsmitte der Linse eingeleitet wird. Beim weiteren Zusammenbewegen der Linsenaufnahmeteile wird die elastische Intraokularlinse gezwungen sich in der Sollposition innerhalb des Linsenaufnahmeraums zu falten oder zu rollen. Gleichzeitig werden die Anschlagflächen an den Linsenhalteteilen aus einer oberen oder äußeren Position in die untere oder innere Endposition, bei welcher die Anschlagsflächen zusammen mit den an den Linsenaufnahmeteilen gebildeten Begrenzungsflächen das Linsenaufnahmefach vorzugsweise bündig umschließen, bewegt. Dabei wird auf die Intraokularlinse von den diametral liegenden Rändern her nach unten in den gefalteten oder gerollten Zustand geschoben.

Die beiden Halteelemente sind federelastisch ausgebildet und sind an Flügeln, mit denen die Linsenaufnahmeteile gegeneinander beim Faltvorgang verschwenkt werden, befestigt. Hierzu können an den innenliegenden Flächen der Flügel Befestigungsstellen für die Halteelemente beispielsweise durch Formschluss, Klemmsitz, Scharniere oder dergleichen vorgesehen sein. Zwischen diesen Befestigungsstellen und den Anschlagflächen weisen die Halteelemente vorzugsweise gebogene Teile auf, welche die Federelastizität der Haltelemente bewirken. Beim Aufeinanderzubewegen der Flügel werden auch die gebogenen Teile der Halteelemente aufeinander zu bewegt und berühren sich. Beim weiteren Schließen der Flügel werden die gebogenen Teile zu linearen Teilen gestreckt, wobei die Anschlagflächen an den Halteelementen die oben erläuterte Bewegung der Anschlagflächen aus einer äußeren Position in eine innere Position bewirken. Die Anschlagflächen sind hierzu vorzugsweise in Ausnehmungen an den Flügeln und/oder der Linsenaufnahmeteile geführt.

An den Linsenaufnahmeteilen oder an den Flügeln sind vorzugsweise in der Nähe des Linsenaufnahmefaches Anschläge vorgesehen, an denen die Halteelemente bei geschlossenem Linsenaufnahmefach aufgestützt sind. An den Halteelementen sind hierzu ebenfalls entsprechende Anschläge vorgesehen. Auf diese Weise lässt sich eine exakte Positionierung der Anschlagflächen bei geschlossenem Linsenaufnahmefach erreichen, so dass eine bündige innere Begrenzungsfläche im Linsenaufnahmefach gebildet wird. Herstellungstoleranzen bei der Herstellung beispielsweise durch Spritzgießen der vorzugsweise aus Kunststoff bestehenden Halteelemente wirken sich nicht nachteilig aus, da der Abstand zwischen den Anschlagflächen und den Anschlägen sehr klein ist. Toleranzen werden im Bereich der gebogenen Teile der Halteelemente oder aufgrund der federelastischen Eigenschaften der Halteelemente ausgeglichen.

Die Linsenaufnahmeteile, welche das Linsenaufnahmefach bilden, sind vorzugsweise als Bestandteile einer Kartusche, welche in einen Injektor eingesetzt werden kann oder am Injektor vorhanden ist, ausgebildet. Das Linsenaufnahmefach ist an seinen in lmplantationsrichtung liegenden Enden offen, wobei das eine Ende in eine Injektorkanüle mündet und durch das andere offene Ende ein Vortriebsstößel, mit welchem die Intraokularlinse in Implantationsrichtung vorgeschoben wird, bewegt werden kann.

Die Kartusche kann so ausgebildet sein, dass die ungefaltet und spannungsfrei in ihr mit Hilfe der Halteinrichtung gelagerte Intraokularlinse sterilisiert und in Bereitschaft beispielsweise für den Versand oder für den Transport gehalten werden kann. Die in der Kartusche liegende Intraokularlinse kann dann durch eine einfache Manipulation, bei welcher die Linsenaufnahmeteile aufeinander zugeschwenkt werden, gefaltet und für die Implantation in den gefalteten oder gerollten Zustand gebracht werden. Die Kartusche dient somit als Vorratsbehälter für die spannungsfrei und ungefaltet in Bereitschaft gehaltene Linse und als Faltvorrichtung für die Linse, um diese in den für die Implantation geeigneten Zustand zu bringen. Für den Transport und/oder die Bereitstellung der Linse kann die Haltevorrichtung oder Kartusche in einem Behälter oder einer Hülle steril angeordnet werden.

Anhand der Figuren wird an Ausführungsbeispielen die Erfindung noch näher erläutert. Es zeigt:
- Fig. 1: in perspektivischer Darstellung ein Ausführungsbeispiel der Erfindung, welches ein als Kartusche ausgebildetes Linsenaufnahmefach als Bestandteil eines Linseninjektors darstellt;
- Fig. 2: an Linsenaufnahmeteilen des Linsenaufnahmefaches der Fig. 1 zu befestigende Halteelemente in perspektivischer Darstellung;
- Fig. 3: in schnittbildlicher Darstellung eine Ausgangsposition des Linsenaufnahmefaches zum Falten der Intraokularlinse;
- Fig. 4: eine Zwischenposition der Bestandteile des Linsenaufnahmefaches während des Faltvorgangs;
- Fig. 5: die Endposition der Bestandteile des Ausführungsbeispiels mit im Linsenaufnahmefach gefalteter Linse;
- Fig. 6: ein weiteres Ausführungsbeispiel der Erfindung, welches ebenfalls als Kartusche ausgebildet ist, in perspektivischer Darstellung und geöffnetem Zustand;
- Fig. 7: in perspektivischer Darstellung ein Halteelement, von welchem zwei im Ausführungsbeispiel der Fig. 6 einzubauen sind;
- Fig. 8: in zusammengebautem Zustand das Ausführungsbeispiel der Erfindung, bei welchem die in den Figuren 6 und 7 dargestellten Bestandteile verwendet sind mit Blickrichtung in das Innere des Linsenaufnahmefaches; und
- Fig. 9: das in Fig. 8 dargestellte Ausführungsbeispiel in perspektivischer Darstellung an der Rückseite.

In den Figuren sind Ausführungsbeispiele einer Vorrichtung zum Falten oder Rollen einer Intraokularlinse 1 dargestellt. Die Ausführungsbeispiele beinhalten ein Linsenaufnahmefach 2, welches von zwei Linsenaufnahmeteilen 3, 4 gebildet wird. Die Linsenaufnahmeteile 3, 4 sind schwenkbar miteinander verbunden. Die Linsenaufnahmeteile 3, 4 sind als Zylindersegmente ausgebildet und in einer längsverlaufenden Schwenkachse schwenkbar miteinander verbunden.

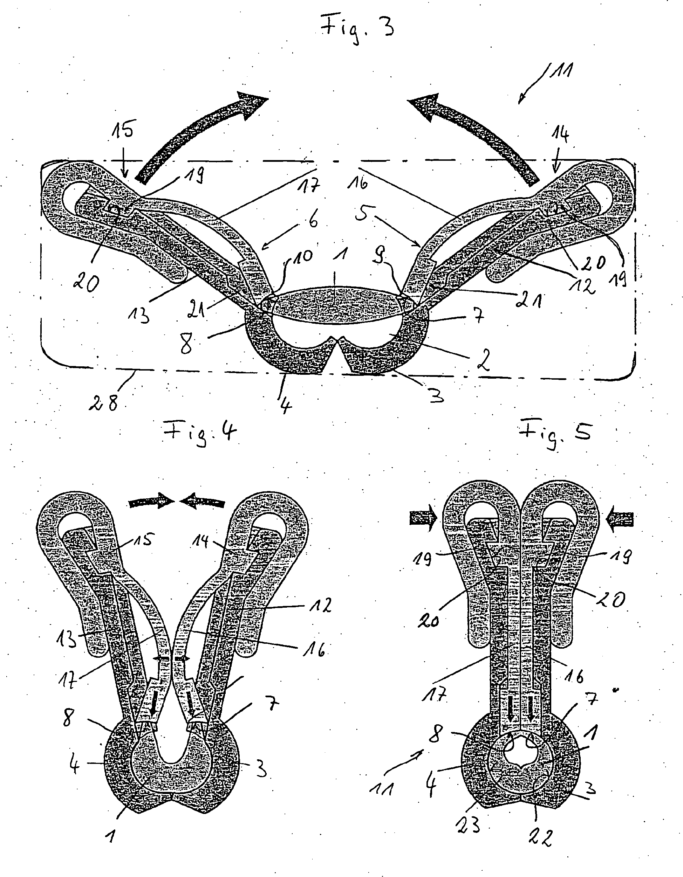

An den Linsenaufnahmeteilen 3, 4 sind Flügel 12, 13 befestigt oder einstückig vorgesehen. Beim Falten der Intraokularlinse 1 werden die Flügel 12, 13 aufeinander zu bewegt, wie es in Fig. 3 durch die aufeinander zugerichteten Pfeile dargestellt ist. Dabei wird die zunächst ungefaltete und spannungsfrei im Linsenaufnahmefach 2 befindliche Intraokularlinse 1 gefaltet, wie im Einzelnen noch anhand der Figuren 4 und 5 erläutert wird.

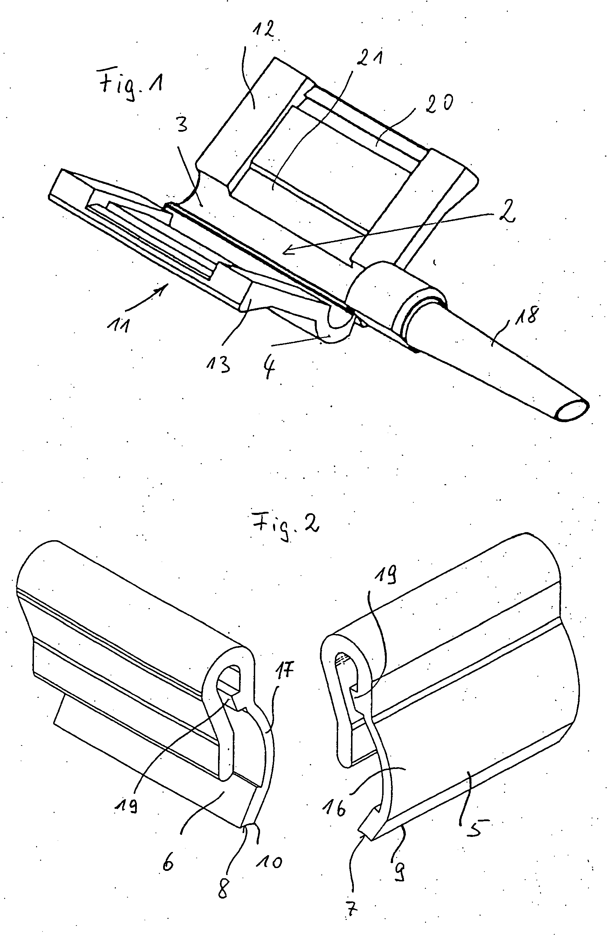

Die Halteelemente, welche, wie aus Fig. 2 zu ersehen ist, die Form von Klammern aufweisen können, sind in Befestigungsstellen 14, 15 an den Flügeln 12, 13 befestigt. Die Befestigung erfolgt durch Formschluss. Hierzu können Vorsprünge 19 an den Haltelementen 5, 6 vorgesehen sein, die mit Formschluss in Ausnehmungen 20 an den Innenflächen der Flügel 12, 13 ragen.

Die Haltelemente besitzen an ihren, dem Linsenaufnahmefach 2 zugekehrten Enden Anschlagflächen 7 und 8. Die mit diesen Anschlagflächen 7, 8 ausgestatteten Haltelemente bilden eine Halteeinrichtung für die im Linsenaufnahmefach 2 angeordnete Intraokularlinse 1. In der in Fig. 3 schematisch dargestellten Ausgangsposition befindet sich die ungefaltete Intraokularlinse 1 spannungsfrei im Linsenaufnahmefach 2. Die Anschlagsflächen 7, 8 liegen über diametralen Randbereichen der Intraokularlinse 1 und verhindern ein Herausfallen der Linse bei der Anordnung der Fig. 3 nach oben hin. Bei der in der Fig. 3 dargestellten Ausgangsposition liegen die diametralen Randbereiche der Linse an der Unterseite auf Flächen der Linsenaufnahmeteile 3 und 4 auf. Diese Auflageflächen bilden mit den Anschlagflächen 7, 8 im Querschnitt etwa dreieckförmige längsverlaufene Räume oder Rinnen zur Aufnahme der diametralen Linsenrandbereiche. Zusätzlich kann die Intraokularlinse 1 auch an der Unterseite entlang ihrer Längsmitte durch die Aufnahmeteile 3, 4 etwa im Bereich der Schwenkachse, mit der diese Teile miteinander verbunden sind, abgestützt werden. Hierdurch wird eine sichere Positionierung der ungefalteten Linse im Linsenaufnahmefach 2 gewährleistet. In der in Fig. 3 dargestellten Anordnung kann die Intraokularlinse 1 sterilisiert und in Bereitschaft gehalten werden. Hierzu kann die in Fig. 3 dargestellte Anordnung in einer Hülle oder einem Behälter 8 in Bereitschaft gehalten oder aufbewahrt werden. Diese Anordnung kann auch transportiert und versandt werden.

Die Halteelemente 5, 6 sind federelastisch ausgebildet und können hierzu zwischen den Anschlagflächen 7, 8 und den Befestigungsstellen 14, 15 gebogene Teile 16, 17 in Plättchenform aufweisen. Beim Aufeinanderzubewegen der Flügel 12, 13 kommen die gebogenen Teile 16, 17, wie in Fig. 4 dargestellt, miteinander in Berührung. Beim weiteren Aufeinanderzuverschwenken werden die gebogenen Teile 16, 17 in eine gestreckte, geradlinige Form gebracht, wie es in Fig. 5 dargestellt ist. Bei diesem Schwenkvorgang werden die Linsenaufnahmeteile 3, 4 aufeinander zu bewegt und die im Linsenaufnahmefach 2 vorhandene Linse zunächst gestaucht und anschließend gefaltet. Beim anfänglichen Stauchen der Linse kommen innenliegende Kanten, welche etwa parallel zur Längsmitte der Intraokularlinse 1 verlaufen in Berührung mit der obenliegenden Linsenfläche. Diese innenliegenden Kanten 9, 10 haben einen Abstand von etwa 1,0 mm vom jeweiligen Linsenrand. Beim Aufeinanderzubewegen der Flügel 12, 13 drücken die Kanten 9, 10 auf die Oberseite der Intraokularlinse 1, so dass diese entlang ihrer Längsmitte nach unten vorgefaltet wird. Beim weiteren Zusammendrücken der Flügel 12, 13, wird wie in Fig. 4 dargestellt, die Linse in Richtung zum Boden des Linsenaufnahmefaches 2 gedrückt und in die gefaltete oder gerollte Form, welche in Fig. 5 dargestellt ist, gebracht. Dabei wird von den Anschlagflächen 7, 8 auf die diametralen Ränder der Linse ein Druck ausgeübt, welcher während der Phase entsteht, bei welcher die gebogenen Teile 16, 17 der Haltelemente 5, 6 in die geradlinige gestreckte Form der Fig. 5 gebracht werden. Dabei bewegen sich die Anschlagflächen 7, 8 aus der innerhalb von Vertiefungen 21 in den Linsenaufnahmeteilen 3, 4 oder den Flügeln 12, 13 zurückgezogenen oder äußeren Positionen (Fig. 3) in Richtung zum Inneren des Linsenaufnahmefaches hin und gelangen in die in Fig. 5 dargestellte Endposition. In dieser Endposition bilden die vorzugsweise konkav ausgebildeten Anschlagsflächen 7, 8 zusammen mit Begrenzungsflächen 23 der Linsenaufnahmeteile 3, 4 eine bündige, zylindrische Begrenzungsfläche des Linsenaufnahmefaches 2, in welchem die gefaltete oder gerollte Linse 1 sich befindet. Der zylindrische Querschnitt kann eine Kreisform oder auch eine hiervon abweichende Form je nach Linsentyp haben.

Das Linsenaufnahmefach kann, wie beim Ausführungsbeispiel dargestellt ist, Bestandteil einer Kartusche 11 sein, die einen Teil eines Injektors mit einer Injektorkanüle 18 bilden. Diese Kartusche 11 kann in den Injektor eingesetzt werden oder aus einem Stück mit dem Injektor gebildet sein. Das Linsenaufnahmefach 2 ist hierzu in Implantationsrichtung an seinen beiden Enden geöffnet. Die eine Öffnung mündet in die Injektorkanüle 18 und die am anderen Ende befindlichen Öffnung bildet einen Zugang für einen nicht näher dargestellten Injektorstößel, mit welchem die im Linsenaufnahmefach befindliche gefaltete oder gerollte Linse durch die Injektorkanüle 18 in das Auge beispielsweise bei einer Kataraktoperation implantiert wird. Beispiele für einen geeigneten Injektorstößel und Injektor sind aus EP 1 438 929 A1 bekannt.

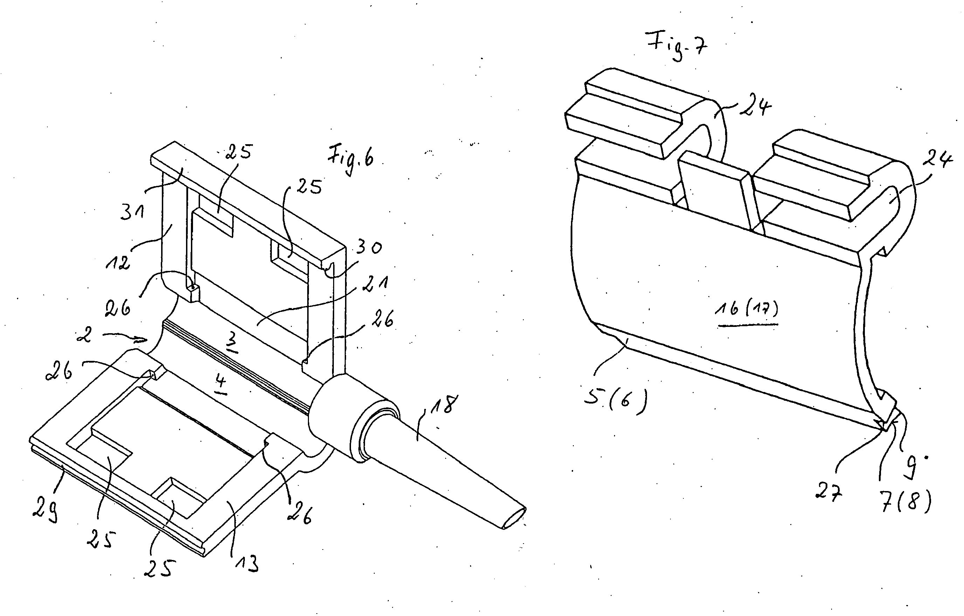

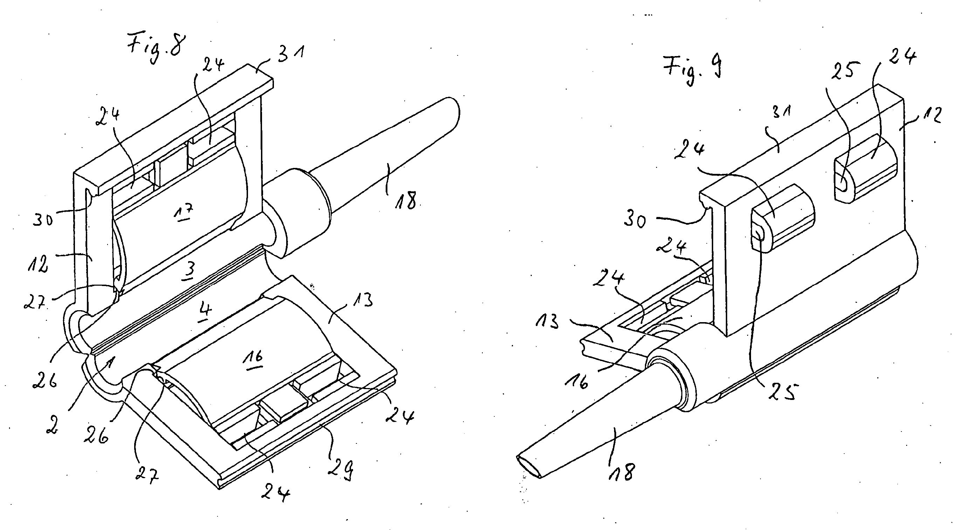

Bei dem in den Figuren 6 bis 9 dargestellten Ausführungsbeispiel werden für gleiche oder gleichwirkende Bauteile die selben Bezugsziffern verwendet, wie im vorher beschriebenen Ausführungsbeispiel. Das in den Figuren 6 bis 9 beschriebene Ausführungsbeispiel weist die Besonderheit auf, dass an den Haltelementen 5 und 6 in unmittelbarer Nähe der Anschlagflächen 7 und 8 Anschläge 27 vorgesehen sind. Diese Anschläge 27 liegen bei geschlossenen Linsenaufnahmeteilen, d.h. bei dem in Fig. 5 dargestellten Zustand an Anschlägen 26 an, die in unmittelbarer Nähe des Linsenaufnahmefaches 2 an den Linsenaufnahmeteilen 3, 4 oder den innenliegenden Enden der Flügel 12, 13 vorgesehen sind. Hierdurch wird die exakte Positionierung der Anschlagflächen 7, 8, welche Bestandteile der den Innenraum des Linsenaufnahmefaches umgrenzenden Begrenzungsfläche sind, bestimmt. Herstellungstoleranzen der Halteelemente 5, 6 wirken sich auch auf den kurzen Abstand zwischen den Anschlagsflächen 7, 8 und den Anschlägen 26, 27 nicht aus. Es wird, wie in Fig. 5 dargestellt ist, eine bündige innenliegende Begrenzungsfläche des geschlossenen Linsenaufnahmefaches 2 zusammen mit den Begrenzungsflächen 22, 23 der Linsenaufnahmeteile erreicht.

Beim Ausführungsbeispiel der Figuren 6 bis 9 erfolgt die Befestigung der Halteelemente 5, 6 mithilfe von U-förmig gebogenen Enden der Halteelemente 5, 6. Diese U-förmig gebogenen Enden 24 sind in Öffnungen 25 der Flügel 12, 13 eingesteckt. Hierdurch werden an jedem Flügel 12, 13 zwei Klemmsitze gebildet, mit denen die Halteelemente 5, 6 an ihren oberen Enden an den Flügeln 12, 13 befestigt sind.

Ferner ist beim Ausführungsbeispiel der Figuren 6 bis 9 am oberen Ende des Flügels 12 ein Raststeg 31 vorgesehen, welcher in Richtung auf den anderen Flügel 13 vorspringt. Am Raststeg 31 ist an der Unterseite ein Rastvorsprung 30 vorgesehen, welcher bei geschlossenem Linsenaufnahmefach 2 in eine Rastnut 29 an der freien Endkante des Flügels 13 eingreift. Beim dargestellten Ausführungsbeispiel sind der Rastvorsprung 30 und die Rastnut 29 als durchgehende sich über die gesamte Länge der Flügel 12, 13 erstreckende Bauelemente ausgebildet. Es ist jedoch auch möglich, mehrere Rastvorsprünge und Rastnuten an den Flügeln 12, 13 vorzusehen. Im geschlossenen Zustand des Linsenaufnahmefaches 2, welches auch beim Ausführungsbeispiel der Figuren 6 bis 9 Bestandteil einer Kartusche ist, erreicht man einen Rastverschluss für die Kartusche, wodurch die Handhabung erleichtert wird. Um das Ausführungsbeispiel der Figuren 6 bis 9 von der geöffneten Stellung in die geschlossene Stellung zu bringen, werden die gleichen Schritte durchgeführt, wie sie anhand der Figuren 3 bis 5 erläutert wurden.

### Bezugszeichenliste

- 1: Intraokuklarlinse
- 2: Linsenaufnahmefach
- 3, 4: Linsenaufnahmeteile
- 5, 6: Haltelemente
- 7, 8: Anschlagflächen
- 9, 10: innenliegende Kanten
- 11: Kartusche
- 12, 13: Flügel
- 14, 15: Befestigungsstellen
- 16, 17: gebogene Teile
- 18: Injektionskanüle
- 19: Vorsprünge
- 20: Ausnehmungen
- 21: Vertiefungen
- 22, 23: Begrenzungsflächen
- 24: U-förmig gebogene Enden der Halteelemente
- 25: Öffnungen in den Flügeln
- 26, 27: Anschläge
- 28: Behälter oder Hülle
- 29: Rastnut
- 30: Rastvorsprung
- 31: Raststeg

## Patentansprüche

1. Vorrichtung zum Falten oder Rollen einer in ein Auge zu implantierenden Intraokularlinse (1) mit einem Linsenaufnahmefach (2), welches von zwei schwenkbar miteinander verbundenen und Flügel (12, 13) aufweisenden Linsenaufnahmeteilen (3, 4) gebildet ist, wobei das Linsenaufnahmefach (2) aus einer offenen Stellung zur Aufnahme der Linse (1) in ungefaltetem Zustand in eine geschlossene Stellung zum Falten oder Rollen der Linse (1) durch Verschwenken der Linsenaufnahmeteile (3, 4) zu bringen ist, und mit einer an den Linsenaufnahmeteilen (3, 4) befestigten Halteeinrichtung zum Halten der Linse (1) bei offener Stellung des Linsenaufnahmefaches (2) und beim Verschwenken der Linsenaufnahmeteile (3, 4), wobei die Halteeinrichtung zwei Haltelemente (5, 6) aufweist, von denen das eine Haltelement (5) am einen Linsenaufnahmeteil (3) und das andere Haltelement (6) am anderen Linsenaufnahmeteil (4) befestigt sind, und mit an den Halteelementen (5, 6) jeweils vorgesehene Anschlagsflächen (7, 8) für diametrale Randbereiche der im offenen Linsenaufnahmefach (2) liegenden ungefalteten Linse (1), wobei bei geschlossenem Aufnahmefach (2) die Anschlagflächen (7, 8) Begrenzungsflächen (22, 23) des Linsenaufnahmefachs (2) bilden, **dadurch gekennzeichnet, dass** die Halteelemente (5, 6) an den Flügeln (12, 13) der Linsenaufnahmeteile (3, 4) federnd beweglich befestigt sind.

2. Vorrichtung nach Anspruch 1, **dadurch gekennzeichnet, dass** die Linsenaufnahmeteile (3, 4) derart ausgebildet sind, dass beim Verschwenken durch elastische Verformung der Haltelemente (5, 6) ein Druck von den Anschlagflächen (7, 8) auf die diametralen Randbereiche der Linse (1) ausgeübt wird.

3. Vorrichtung nach Anspruch 1 oder 2, **dadurch gekennzeichnet, dass** an den Anschlagflächen (7, 8) innenliegende Kanten (9, 10) ausgebildet und geeignet sind, an der zu den Kanten (9, 10) im Wesentlichen parallel verlaufenden Längsmitte der Linse (1) den Faltvorgang einzuleiten.

4. Vorrichtung nach einem der Ansprüche 1 bis 3, **dadurch gekennzeichnet, dass** die Anschlagflächen (7, 8) zwischen einer äußeren Position bei offenem Linsenaufnahmefach (2) und einer innern Position bei geschlossenem Linsenaufnahmefach (2) beweglich geführt sind.

5. Vorrichtung nach Anspruch 4, **dadurch gekennzeichnet, dass** die Haltelemente (5, 6) im Bereich der freien Enden der Flügel (12, 13) in Befestigungsstellen (14, 15) befestigt sind und zwischen den Befestigungsstellen (14, 15) und den Anschlagflächen (7, 8) federnd elastisch gebogene Teile (16, 17) an den Haltelementen (5, 6) vorgesehen sind.

6. Vorrichtung nach einem der Ansprüche 1 bis 5, **dadurch gekennzeichnet, dass** die Halteelemente (5, 6) mittels Formschluss (19, 20) oder Klemmsitz (24, 25) oder Scharnieren an den Linsenaufnahmeteilen (3, 4) oder den Flügeln (12, 13) befestigt sind.

7. Vorrichtung nach einem der Ansprüche 1 bis 6, **dadurch gekennzeichnet, dass** die Anschlagflächen (7, 8) konkav ausgebildet sind.

8. Vorrichtung nach einem der Ansprüche 1 bis 7, **dadurch gekennzeichnet, dass** die gebogenen Teile (16, 17) der Haltelemente (5, 6) beim Bewegen in die geschlossene Stellung des Aufnahmefaches (2) in gestreckte geradlinige Positionen bei geschlossenem Linsenaufnahmefach (2) bewegt sind und über ihre Anschlagflächen (7, 8) auf die zu faltende oder zu rollende Linse (1) einen Druck ausüben.

9. Vorrichtung nach einem der Ansprüche 1 bis 6, **dadurch gekennzeichnet, dass** die Haltelemente (5, 6) als Klammern ausgebildet sind.

10. Vorrichtung nach einem der Ansprüche 1 bis 9, **dadurch gekennzeichnet, dass** die Befestigungsstellen (14, 15)an den aufeinander zu gerichteten Seiten der Flügel (12, 13) vorgesehen sind.

11. Vorrichtung nach einem der Ansprüche 1 bis 10, **dadurch gekennzeichnet, dass** die Haltelemente (5, 6) zumindest im Bereich der Anschlagsflächen (7, 8) an den Flügeln (12, 13) und oder den Linsenaufnahmeteilen (3, 4) beweglich geführt sind.

12. Vorrichtung nah einem der Ansprüche 1 bis 11, **dadurch gekennzeichnet, dass** das Linsenaufnahmefach (2) Bestandteil einer Kartusche (11) ist, wobei das Linsenaufnahmefach (2) in seiner geschlossenen Stellung an seinen in Implantationsrichtung liegenden Enden offen ist.

13. Vorrichtung nach einem der Ansprüche 1 bis 12, **dadurch gekennzeichnet, dass** das eine offene Ende des Linsenaufnahmefaches (2) in eine Injektionskanüle mündet und das andere offene Ende des Linsenaufnahmefaches (2) einen Zugang für einen Vorschubstößel bildet, mit welchem die im Linsenaufnahmefach (2) angeordnete gerollte oder gefaltete Linse durch die Injektorkanüle (18) zu schieben ist.

14. Vorrichtung nach einem der Ansprüche 1 bis 13, **dadurch gekennzeichnet, dass** an den Linsenaufnahmeteilen (3, 4) oder den Flügeln (12, 13) Anschläge (26) vorgesehen sind, an denen die Halteelemente (5, 6) bei geschlossenem Linsenaufnahmefach (2) abgestützt sind.

15. Vorrichtung nach Anspruch 14, **dadurch gekennzeichnet, dass** die Anschläge (26) in der Nähe des Linsenaufnahmefaches (2) vorgesehen sind.

16. Vorrichtung nach einem der Ansprüche 1 bis 15, **dadurch gekennzeichnet, dass** die in ungefaltetem und spannungsfrei im Linsenaufnahmefach (2) angeordnete und seitlich an den Anschlagflächen (7, 8) gehaltene Intraokularlinse geeignet ist, in einem Behälter oder einer Hülle (28) in Bereitschaft gehalten zu werden.

17. Vorrichtung nach einem der Ansprüche 1 bis 16, **dadurch gekennzeichnet, dass** die Linsenaufnahmeteile (3, 4) mittels eines Verschlusses (29, 31), insbesondere Rastverschlusses im geschlossenen Zustand gehalten sind.

18. Vorrichtung nach Anspruch 17, **dadurch gekennzeichnet, dass** der Verschluss (29, 31) an den freien Enden der Flügel (12, 13) vorgesehen ist.

## Claims

1. Apparatus for folding or rolling an intraocular lens (1) to be implanted into an eye, comprising a lens reception compartment (2) formed by two lens reception members (3, 4) which are rotatably connected to each other and comprise wings (12, 13), wherein the lens reception compartment (2) is capable of being transformed from an opened position for receiving the lens (1) in an unfolded status to a closed position for folding or rolling the lens (1) by pivoting the lens reception members (3, 4), and a holding device attached to the lens reception members (3, 4) for holding the lens (1) while the lens reception compartment (2) being in the open position and while pivoting the lens reception members (3, 4), wherein the holding device comprises two holding members (5, 6) of which one holding member (5) being attached to the one lens reception member (3) and the other holding element (6) being attached to the other lens reception member (4), and stop surfaces (7, 8) respectively provided at the holding members (5, 6) for diametrical boundary areas of the unfolded lens (1) lying in the opened lens reception compartment (2), wherein the stop surfaces (7, 8) form boundary surfaces (22, 23) of the lens reception compartment (2) when the reception compartment (2) is closed,
**characterized in that** the holding members (5, 6) are attached to the wings (12, 13) of the lens reception members (3, 4) in a resilient movable manner.

2. Apparatus according to claim 1, **characterized in that** the lens reception members (3, 4) are formed such that by means of resilient deformation of the holding members (5, 6) a pressure is applied from the stop surfaces (7, 8) to the diametrical boundary areas of the lens (1) during pivoting.

3. Apparatus according to claim 1 or 2, **characterized in that** internal edges (9, 10) are formed at stop surfaces (7, 8) and being suitable to initiate the folding operation at the longitudinal center of the lens (1) extending substantially parallel to the edges (9, 10).

4. Apparatus according to one of the claims 1 to 3, **characterized in that** the stop surfaces (7, 8) being movably guided between an outer position when the lens reception compartment (2) is open and an inner position when the lens reception compartment (2) is closed.

5. Apparatus according to claim 4, **characterized in that** the holding members (5, 6) are fixed in fixing locations (14, 15) in the area of the free ends of the wings (12, 13), and members (16, 17) resiliently bent between the fixing locations (14, 15) and the stop surfaces (7, 8) are provided at the holding members (5, 6).

6. Apparatus according to one of the claims 1 to 5, **characterized in that** the holding members (5, 6) are fixed to the lens reception members (3, 4) or to the wings (12, 13) by means of form fit (19, 20) or force fit (24, 25) or hinge-joints.

7. Apparatus according to one of the claims 1 to 6, **characterized in that** the stop surfaces (7, 8) are formed concave.

8. Apparatus according to one of the claims 1 to 7, **characterized in that**, when moving to the closed position of the reception compartment (2), the bent members (16, 17) of the holding members (5, 6) are moved to elongated straight positions when the lens reception compartment (2) is closed and apply via their stop surfaces (7, 8) a pressure to the lens (1) to be folded or rolled.

9. Apparatus according to one of the claims 1 to 6, **characterized in that** the holding members (5, 6) are formed as clamps.

10. Apparatus according to one of the claims 1 to 9, **characterized in that** the fixing locations (14, 15) are provided at sides of the wings (12, 13) facing each other.

11. Apparatus according to one of the claims 1 to 10, **characterized in that** the holding members (5, 6) being movably guided at least in the area of the stop surfaces (7, 8) at the wings (12, 13) and/or the lens reception members (3, 4).

12. Apparatus according to one of the claims 1 to 11, **characterized in that** the lens reception compartment (2) is a part of a cartridge (11), wherein the lens reception compartment (2) in its closed position is open at its ends facing in implanting direction.

13. Apparatus according to one of the claims 1 to 12, **characterized in that** the one open end of the lens reception compartment (2) opens out into an injection cannula, and the other open end of the lens reception compartment (2) forms an access for a traverse rod with which the rolled or folded lens arranged in the lens reception compartment (2) is to be pushed through the injector cannula (18).

14. Apparatus according to one of the claims 1 to 13, **characterized in that** stops (26) are provided at the lens reception members (3, 4) or the wings (12, 13), on which stops (26) the holding members (5, 6) are supported when the lens reception compartment (2) is closed.

15. Apparatus according to claim 14, **characterized in that** the stops (26) are provided in proximity to the lens reception compartment (2).

16. Apparatus according to one of the claims 1 to 15, **characterized in that** the intraocular lens, which is arranged in the lens reception compartment (2) in an unfolded and strainless state and laterally held at the stop surfaces (7, 8), is adapted to be held in a receptacle or a casing (28).

17. Apparatus according to one of the claims 1 to 16, **characterized in that** the lens reception members (3, 4) are held in the closed state by means of a closure (29, 31), in particular a snapping closure.

18. Apparatus according to claim 17, **characterized in that** the closure (29, 31) is provided at the free ends of the wings (12, 13).

## Revendications

1. Dispositif pour plier ou enrouler une lentille intraoculaire (1) à implanter dans un oeil, avec un réceptacle de lentille (2) formé par deux parties de réception de lentille (3, 4) reliées ensemble de manière pivotable et présentant des volets (12, 13), dans lequel le réceptacle de lentille (2) peut être amené d'une position ouverte pour recevoir la lentille (1) à l'état non plié dans une position fermée pour plier et enrouler la lentille (1) par un pivotement des parties de réception de lentille (3, 4), et avec un dispositif de retenue, fixé aux parties de réception de lentille (3, 4) pour retenir la lentille (1) dans la position ouverte du réceptacle de lentille (2) et lors du pivotement des parties de réception de lentille (3, 4), dans lequel le dispositif de retenue présente deux éléments de retenue (5, 6), dont un élément de retenue (5) est fixé à une partie de réception de lentille (3) et l'autre élément de retenue (6) est fixé à l'autre partie de réception de lentille (4), et avec des surfaces de butée (7, 8) prévues respectivement au niveau des éléments de retenue (5, 6) pour des zones de bord diamétrales de la lentille (1) non pliée posée dans le réceptacle de lentille (2) ouvert, dans lequel, lorsque le réceptacle (2) est fermé, les surfaces de butée (7, 8) constituent des surfaces limites (22, 23) du réceptacle de lentille (2), **caractérisé en ce que** les éléments de retenue (5, 6) sont fixés aux volets (12, 13) des parties de réception de lentille (3, 4) de façon élastiquement mobile.

2. Dispositif selon la revendication 1, **caractérisé en ce que** les parties de réception de lentille (3, 4) sont réalisées de telle sorte qu'en cas de pivotement, une déformation élastique des éléments de retenue (5, 6) exerce une pression à partir des surfaces de butée (7, 8) sur les zones de bord diamétrales de la lentille (1).

3. Dispositif selon la revendication 1 ou 2, **caractérisé en ce que** des arêtes intérieures (9, 10) sont réalisées sur les surfaces de butée (7, 8) et adaptées pour amorcer l'opération de pliage au niveau du centre longitudinal de la lentille (1) s'étendant substantiellement en parallèle aux arêtes (9, 10).

4. Dispositif selon l'une quelconque des revendications 1 à 3, **caractérisé en ce que** les surfaces de butée (7, 8) sont guidées de façon mobile entre une position extérieure lorsque le réceptacle de lentille (2) est ouvert et une position intérieure lorsque le réceptacle de lentille (2) est fermé.

5. Dispositif selon la revendication 4, **caractérisé en ce que** les éléments de retenue (5, 6) sont fixés dans la zone des extrémités libres des volets (12, 13) à des points de fixation (14, 15), et entre les points de fixation (14, 15) et les surfaces de butée (7, 8), des parties (16, 17) courbées de façon élastique à la manière d'un ressort sont prévues sur les éléments de retenue (5, 6).

6. Dispositif selon l'une quelconque des revendications 1 à 5, **caractérisé en ce que** les éléments de retenue (5, 6) sont fixés aux parties de réception de lentille (3, 4) ou aux volets (12, 13) par complémentarité de forme (19, 20) ou par ajustement serré (24, 25) ou par des charnières.

7. Dispositif selon l'une quelconque des revendications 1 à 6, **caractérisé en ce que** les surfaces de butée (7, 8) sont réalisées de façon concave.

8. Dispositif selon l'une quelconque des revendications 1 à 7, **caractérisé en ce que** les parties courbes (16, 17) des éléments de retenue (5, 6), lors du déplacement vers la position fermée du réceptacle (2), sont déplacées vers des positions rectilignes allongées lorsque le réceptacle de lentille (2) est fermé et exercent par leurs surfaces de butée (7, 8) une pression sur la lentille (1) à plier ou à enrouler.

9. Dispositif selon l'une quelconque des revendications 1 à 6, **caractérisé en ce que** les éléments de retenue (5, 6) sont réalisés sous forme d'attaches.

10. Dispositif selon l'une quelconque des revendications 1 à 9, **caractérisé en ce que** les points de fixation (14, 15) sont prévus sur les faces des volets (12, 13) tournées les unes vers les autres.

11. Dispositif selon l'une quelconque des revendications 1 à 10, **caractérisé en ce que** les éléments de retenue (5, 6) sont guidés de façon mobile au moins au niveau des surfaces de butée (7, 8) sur les volets (12, 13) et/ou les parties de réception de lentille (3, 4).

12. Dispositif selon l'une quelconque des revendications 1 à 11, **caractérisé en ce que** le réceptacle de lentille (2) fait partie intégrante d'une cartouche (11), dans lequel le réceptacle de lentille (2), dans sa position fermée, est ouvert au niveau de ses extrémités situées dans la direction d'implantation.

13. Dispositif selon l'une quelconque des revendications 1 à 12, **caractérisé en ce que** ladite extrémité ouverte du réceptacle de lentille (2) donne sur une canule d'injection et l'autre extrémité ouverte du réceptacle de lentille (2) constitue un accès pour un piston d'avance, avec lequel il convient de pousser la lentille enroulée ou pliée, disposée dans le réceptacle de lentille (2), à travers la canule d'injection (18).

14. Dispositif selon l'une quelconque des revendications 1 à 13, **caractérisé en ce qu'**aux parties de réception de lentille (3, 4) ou aux volets (12, 13), des butées (26) sont prévues, sur lesquelles s'appuient les éléments de retenue (5, 6) lorsque le réceptacle de lentille (2) est fermé.

15. Dispositif selon la revendication 14, **caractérisé en ce que** les butées (26) sont prévues à proximité du réceptacle de lentille (2).

16. Dispositif selon l'une quelconque des revendications 1 à 15, **caractérisé en ce que** la lentille intraoculaire, disposée dans le réceptacle de lentille (2) à l'état non plié et détendu, et retenue latéralement contre les surfaces de butée (7, 8), est adaptée pour être tenue à disposition dans un récipient ou une gaine (28).

17. Dispositif selon l'une quelconque des revendications 1 à 16, **caractérisé en ce que** les parties de réception de lentille (3, 4) sont tenues à l'état fermé au moyen d'une fermeture (29, 31), en particulier d'une fermeture à cran d'arrêt.

18. Dispositif selon la revendication 17, **caractérisé en ce que** la fermeture (29, 31) est prévue aux extrémités libres des volets (12, 13).
